# EUROPEAN PATENT SPECIFICATION

(11) **EP 2 298 171 B1**
(45) Date of publication and mention of the grant of the patent: **18.03.2015**
(21) Application number: 10186583.0
(22) Date of filing: 03.06.2005
(51) Int. Cl.: A61B 5/151

(54) **Tissue interface on a fluid sampling device**
Gewebeschnittstelle auf einer Fluidprobenvorrichtung
Interface de tissus sur un dispositif d'échantillonnage de fluides

(30) Priority: 03.06.2004 US 577297 P
(43) Date of publication of application: 23.03.2011
(62) Divisional of application: 05783821.1
(73) Proprietor: Sanofi-Aventis Deutschland GmbH, 65929 Frankfurt am Main (DE)
(72) Inventor: Deshmukh, Ajay, Mountain View, CA 94041 (US); Hegener, Jason, H., Palo Alto, CA 94303 (US); Mauze, Ganapati, Sunnyvale, CA 94087 (US); Von Duering, Monika, U., Palo Alto, CA 94303 (US); Freeman, Dominique, M., La Honda, CA 94020 (US)
(74) Representative: Mintz Levin Cohn Ferris Glovsky and Popeo LLP

(56) References cited:
- WO-A-01/95806
- WO-A-2004/045375
- US-B1- 6 589 260

## Description

### BACKGROUND OF THE INVENTION

### Technical Field:

The technical field relates to analyte detecting devices, and more specifically, to sample capture and tissue interface for analyte detecting devices.

### Background Art:

Test strips are known in the medical health-care products industry for analyzing analyte levels such as but not limited to, glucose levels in blood. For this type of analysis, a drop of blood is typically obtained by making a small incision in the fingertip, creating a small wound, which generates a small blood droplet on the surface of the skin. A test strip is brought by the user to the blood droplet at the wound and engaged in a manner to bring blood to an analysis site on the test strip. The test strip is then coupled to a metering device which typically uses an electrochemical technique to determine the amount of glucose in the blood.

Early methods of using test strips required a relatively substantial volume of blood to obtain an accurate glucose measurement. This large blood requirement made the monitoring experience a painful one for the user since the user may need to lance deeper than comfortable to obtain sufficient blood generation. Alternatively, if insufficient blood is spontaneously generated, the user may need to "milk" the wound to squeeze enough blood to the skin surface. Neither method is desirable as they take additional user effort and may be painful. The discomfort and inconvenience associated with such lancing events may deter a user from testing their blood glucose levels in a rigorous manner sufficient to control their diabetes.

A further impediment to patient compliance is the amount of time that it takes for a glucose measurement to be completed. Known devices can take a substantial amount of time to arrive at a glucose level. The more time it takes to arrive at a measurement, the less the likely that the user will stay with their testing regime. A further impediment to patient compliance is the amount of time that at lower volumes, it becomes even more important that blood or other fluid sample be directed to a measurement device without being wasted or spilled along the way. Known devices do not effectively handle the low sample volumes in an efficient manner. Accordingly, improved sensing devices are desired to increase user compliance and reduce the hurdles associated with analyte measurement.

### SUMMARY OF THE INVENTION

The present invention provides solutions for at least some of the drawbacks discussed above. Specifically, some embodiments of the present invention provide an improved apparatus for measuring analyte levels in a body fluid, as defined by claim 1 and the dependent claims.

### BRIEF DESCRIPTION OF THE DRAWINGS

Figure 1 illustrates an embodiment of a controllable force driver in the form of a cylindrical electric penetrating member driver using a coiled solenoid -type configuration.
Figure 2A illustrates a displacement over time profile of a penetrating member driven by a harmonic spring/mass system.
Figure 2B illustrates the velocity over time profile of a penetrating member driver by a harmonic spring/mass system.
Figure 2C illustrates a displacement over time profile of an embodiment of a controllable force driver.
Figure 2D illustrates a velocity over time profile of an embodiment of a controllable force driver.
Figure 3 is a diagrammatic view illustrating a controlled feed-back loop.
Figure 4 is a perspective view of a tissue penetration device having features of the invention.
Figure 5 is an elevation view in partial longitudinal section of the tissue penetration device of Figure 4.
Figure 6 shows a schematic of a penetrating member piercing tissue.
Figure 7 shows an exploded perspective view of one embodiment of a device.
Figure 8 shows a schematic of a penetrating member piercing tissue.
Figure 9 shows the vascular supply of a finger pad.
Figures 10 and 11 show perspective views of one interface.
Figures 12A and 12B show an embodiment according to the present invention.
Figures 13 and 14 show yet another embodiment not included in the present invention.
Figure 15 shows a perspective view of a still further embodiment not included in the present invention.
Figure 16 shows a perspective view of another embodiment not included in the present invention.
Figure 17 shows a perspective view of another embodiment not included in the present invention.
Figure 18 shows one embodiment of a kit.
Figure 19 shows a body fluid sampling device.
Figure 20 shows a cassette.

### DESCRIPTION OF THE SPECIFIC EMBODIMENTS

It is to be understood that both the foregoing general description and the following detailed description are exemplary and explanatory only and are not restrictive of the invention, as claimed. It may be noted that, as used in the specification and the appended claims, the singular forms "a", "an" and "the" include plural referents unless the context clearly dictates otherwise. Thus, for example, reference to "a material" may include mixtures of materials, reference to "a chamber" may include multiple chambers, and the like.

In this specification and in the claims which follow, reference will be made to a number of terms which shall be defined to have the following meanings:

"Optional" or "optionally" means that the subsequently described circumstance may or may not occur, so that the description includes instances where the circumstance occurs and instances where it does not. For example, if a device optionally contains a feature for analyzing a blood sample, this means that the analysis feature may or may not be present, and, thus, the description includes structures wherein a device possesses the analysis feature and structure wherein the analysis feature is not present.

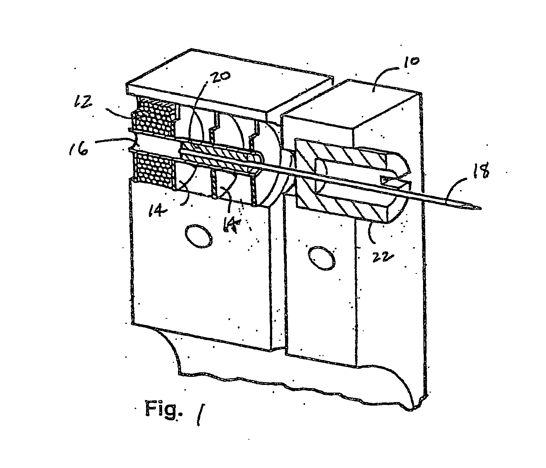

The present invention may be used with a variety of different penetrating member drivers. It is contemplated that these penetrating member drivers may be spring based, solenoid based, magnetic driver based, nanomuscle based, or based on any other mechanism useful in moving a penetrating member along a path into tissue. It should be noted that the present invention is not limited by the type of driver used with the penetrating member feed mechanism. One suitable penetrating member driver for use with the present invention is shown in Figure 1. This is an embodiment of a solenoid type electromagnetic driver that is capable of driving an iron core or slug mounted to the penetrating member assembly using a direct current (DC) power supply. The electromagnetic driver includes a driver coil pack that is divided into three separate coils along the path of the penetrating member, two end coils and a middle coil. Direct current is alternated to the coils to advance and retract the penetrating member. Although the driver coil pack is shown with three coils, any suitable number of coils may be used, for example, 4, 5, 6, 7 or more coils may be used.

Referring to the embodiment of Figure 1, the stationary iron housing 10 may contain the driver coil pack with a first coil 12 flanked by iron spacers 14 which concentrate the magnetic flux at the inner diameter creating magnetic poles. The inner insulating housing 16 isolates the penetrating member 18 and iron core 20 from the coils and provides a smooth, low friction guide surface. The penetrating member guide 22 further centers the penetrating member 18 and iron core 20. The penetrating member 18 is protracted and retracted by alternating the current between the first coil 12, the middle coil, and the third coil to attract the iron core 20. Reversing the coil sequence and attracting the core and penetrating member back into the housing retracts the penetrating member. The penetrating member guide 22 also serves as a stop for the iron core 20 mounted to the penetrating member 18.

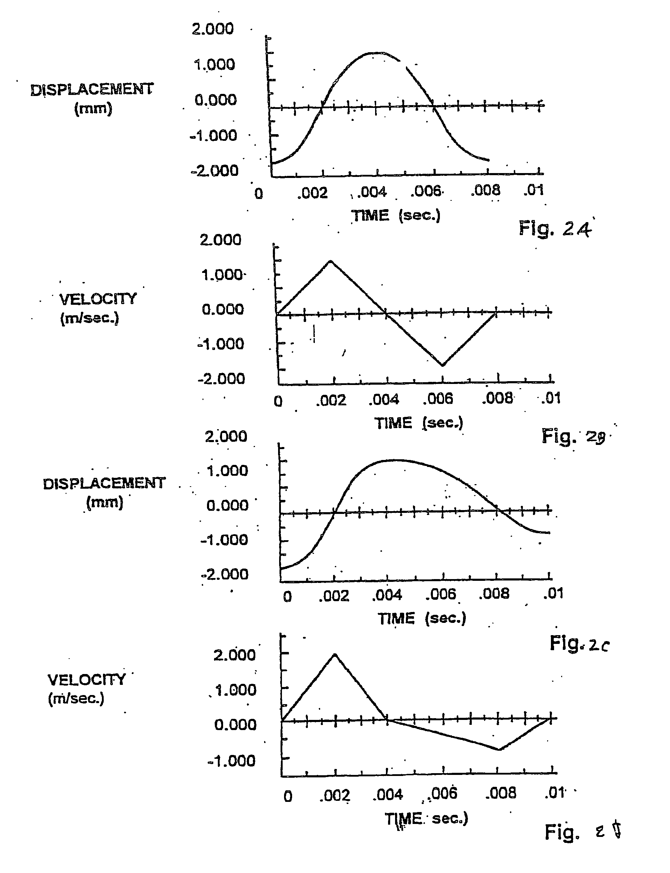

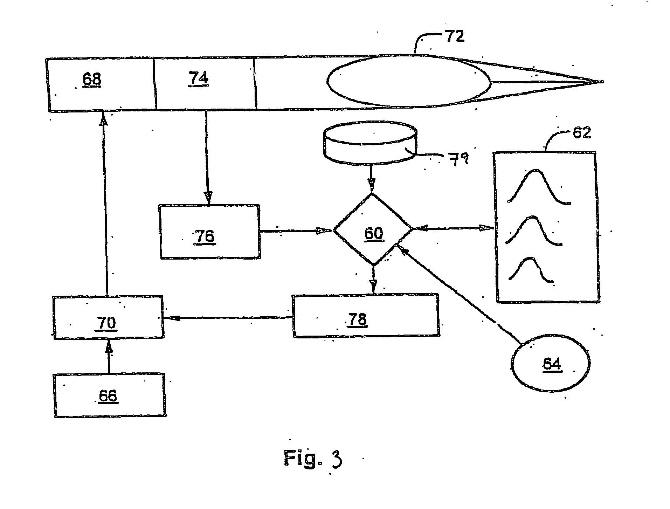

As discussed above, tissue penetration devices which employ spring or cam driving methods have a symmetrical or nearly symmetrical actuation displacement and velocity profiles on the advancement and retraction of the penetrating member as shown in Figures 2 and 3. In most of the available lancet devices, once the launch is initiated, the stored energy determines the velocity profile until the energy is dissipated. Controlling impact, retraction velocity, and dwell time of the penetrating member within the tissue can be useful in order to achieve a high success rate while accommodating variations in skin properties and minimize pain. Advantages can be achieved by taking into account of the fact that tissue dwell time is related to the amount of skin deformation as the penetrating member tries to puncture the surface of the skin and variance in skin deformation from patient to patient based on skin hydration.

In this embodiment, the ability to control velocity and depth of penetration may be achieved by use of a controllable force driver where feedback is an integral part of driver control. Such drivers can control either metal or polymeric penetrating members or any other type of tissue penetration element. The dynamic control of such a driver is illustrated in Figure. 2C which illustrates an embodiment of a controlled displacement profile and Figure 2D which illustrates an embodiment of a the controlled velocity profile. These are compared to Figures 2A and 2B, which illustrate embodiments of displacement and velocity profiles, respectively, of a harmonic spring/mass powered driver. Reduced pain can be achieved by using impact velocities of greater than about 2 m/s entry of a tissue penetrating element, such as a lancet, into tissue. Other suitable embodiments of the penetrating member driver are described in commonly assigned, copending U.S. Patent Application Ser. No. 10/127,395, (Attorney Docket No. 38187-2551) filed April 19, 2002.

Figure 3 illustrates the operation of a feedback loop using a processor 60. The processor 60 stores profiles 62 in non-volatile memory. A user inputs information 64 about the desired circumstances or parameters for a lancing event. The processor 60 selects a driver profile 62 from a set of alternative driver profiles that have been preprogrammed in the processor 60 based on typical or desired tissue penetration device performance determined through testing at the factory or as programmed in by the operator. The processor 60 may customize by either scaling or modifying the profile based on additional user input information 64. Once the processor has chosen and customized the profile, the processor 60 is ready to modulate the power from the power supply 66 to the penetrating member driver 68 through an amplifier 70. The processor 60 may measure the location of the penetrating member 72 using a position sensing mechanism 74 through an analog to digital converter 76 linear encoder or other such transducer. Examples of position sensing mechanisms have been described in the embodiments above and may be found in the specification for commonly assigned, copending U.S. Patent Application Ser. No. 10/127,395, (Attorney Docket No. 38187-2551) filed April 19, 2002. The processor 60 calculates the movement of the penetrating member by comparing the actual profile of the penetrating member to the predetermined profile. The processor 60 modulates the power to the penetrating member driver 68 through a signal generator 78, which may control the amplifier 70 so that the actual velocity profile of the penetrating member does not exceed the predetermined profile by more than a preset error limit. The error limit is the accuracy in the control of the penetrating member.

After the lancing event, the processor 60 can allow the user to rank the results of the lancing event. The processor 60 stores these results and constructs a database 80 for the individual user. Using the database 79, the processor 60 calculates the profile traits such as degree of painlessness, success rate, and blood volume for various profiles 62 depending on user input information 64 to optimize the profile to the individual user for subsequent lancing cycles. These profile traits depend on the characteristic phases of penetrating member advancement and retraction. The processor 60 uses these calculations to optimize profiles 62 for each user. In addition to user input information 64, an internal clock allows storage in the database 79 of information such as the time of day to generate a time stamp for the lancing event and the time between lancing events to anticipate the user's diurnal needs. The database stores information and statistics for each user and each profile that particular user uses.

In addition to varying the profiles, the processor 60 can be used to calculate the appropriate penetrating member diameter and geometry suitable to realize the blood volume required by the user. For example, if the user requires about 1-5 microliter volume of blood, the processor 60 may select a 200 micron diameter penetrating member to achieve these results. For each class of penetrating member, both diameter and penetrating member tip geometry, is stored in the processor 60 to correspond with upper and lower limits of attainable blood volume based on the predetermined displacement and velocity profiles.

The body fluid sampling device is capable of prompting the user for information at the beginning and the end of the lancing event to more adequately suit the user. The goal is to either change to a different profile or modify an existing profile. Once the profile is set, the force driving the penetrating member is varied during advancement and retraction to follow the profile. The method of lancing using the device comprises selecting a profile, lancing according to the selected profile, determining lancing profile traits for each characteristic phase of the lancing cycle, and optimizing profile traits for subsequent lancing events.

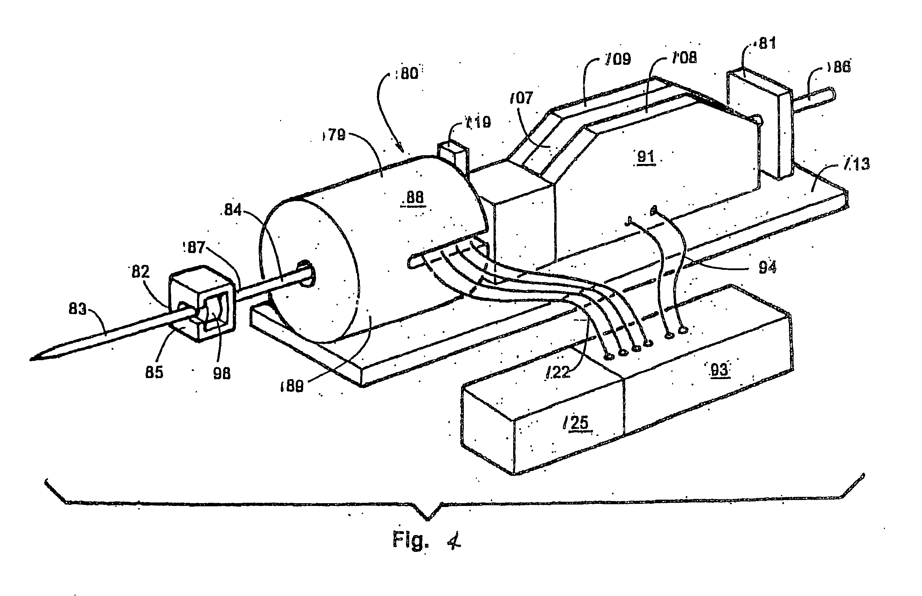

Figure 4 illustrates an embodiment of a tissue penetration device, more specifically, a body fluid sampling device 80 that includes a controllable driver 179 coupled to a tissue penetration element. The body fluid sampling device 80 has a proximal end 81 and a distal end 82. At the distal end 82 is the tissue penetration element in the form of a penetrating member 83, which is coupled to an elongate coupler shaft 84 by a drive coupler 85. The elongate coupler shaft 84 has a proximal end 86 and a distal end 87. A driver coil pack 88 is disposed about the elongate coupler shaft 84 proximal of the penetrating member 83. A position sensor 91 is disposed about a proximal portion 92 of the elongate coupler shaft 84 and an electrical conductor 94 electrically couples a processor 93 to the position sensor 91. The elongate coupler shaft 84 driven by the driver coil pack 88 controlled by the position sensor 91 and processor 93 form the controllable driver, specifically, a controllable electromagnetic driver.

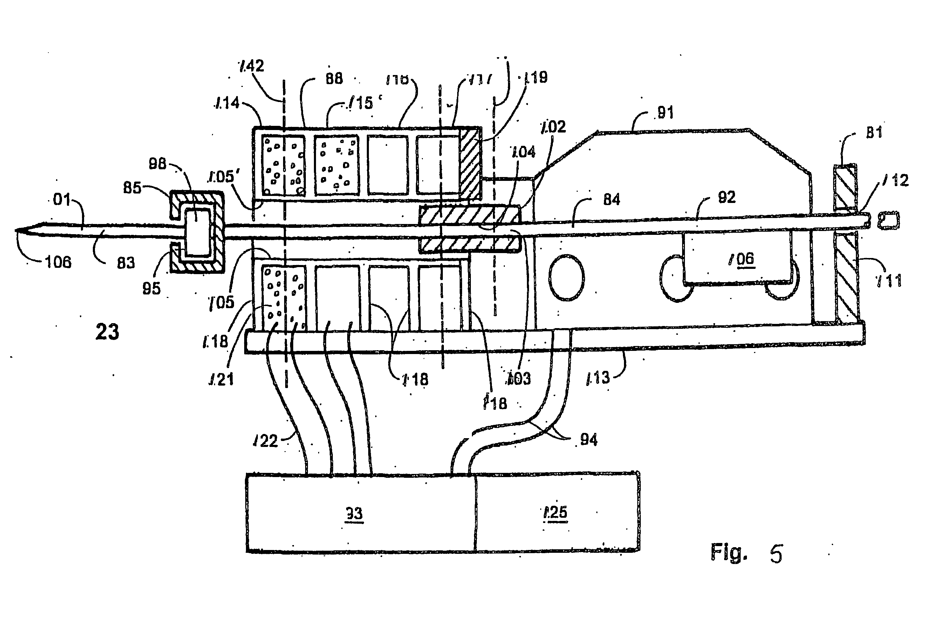

Referring to Figure 5, the body fluid sampling device 80 can be seen in more detail, in partial longitudinal section. The penetrating member 83 has a proximal end 95 and a distal end 96 with a sharpened point at the distal end 96 of the penetrating member 83 and a drive head 98 disposed at the proximal end 95 of the penetrating member 83. A penetrating member shaft 201 is disposed between the drive head 98 and the sharpened point 97. The penetrating member shaft 201 may be comprised of stainless steel, or any other suitable material or alloy and have a transverse dimension of about 0.1 to about 0.4 mm. The penetrating member shaft may have a length of about 3 mm to about 50 mm, specifically, about 15 mm to about 20 mm. The drive head 98 of the penetrating member 83 is an enlarged portion having a transverse dimension greater than a transverse dimension of the penetrating member shaft 201 distal of the drive head 98. This configuration allows the drive head 98 to be mechanically captured by the drive coupler 85. The drive head 98 may have a transverse dimension of about 0.5 to about 2 mm.

A magnetic member 102 is secured to the elongate coupler shaft 84 proximal of the drive coupler 85 on a distal portion 203 of the elongate coupler shaft 84. The magnetic member 102 is a substantially cylindrical piece of magnetic material having an axial lumen 204 extending the length of the magnetic member 102. The magnetic member 102 has an outer transverse dimension that allows the magnetic member 102 to slide easily within an axial lumen 105 of a low friction, possibly lubricious, polymer guide tube 105' disposed within the driver coil pack 88. The magnetic member 102 may have an outer transverse dimension of about 1.0 to about 5.0 mm, specifically, about 2.3 to about 2.5 mm. The magnetic member 102 may have a length of about 3.0 to about 5.0 mm, specifically, about 4.7 to about 4.9 mm. The magnetic member 102 can be made from a variety of magnetic materials including ferrous metals such as ferrous steel, iron, ferrite, or the like. The magnetic member 102 may be secured to the distal portion 203 of the elongate coupler shaft 84 by a variety of methods including adhesive or epoxy bonding, welding, crimping or any other suitable method.

Proximal of the magnetic member 102, an optical encoder flag 206 is secured to the elongate coupler shaft 84. The optical encoder flag 206 is configured to move within a slot 107 in the position sensor 91. The slot 107 of the position sensor 91 is formed between a first body portion 108 and a second body portion 109 of the position sensor 91. The slot 107 may have separation width of about 1.5 to about 2.0 mm. The optical encoder flag 206 can have a length of about 14 to about 18 mm, a width of about 3 to about 5 mm and a thickness of about 0.04 to about 0.06 mm.

The optical encoder flag 206 interacts with various optical beams generated by LEDs disposed on or in the position sensor body portions 108 and 109 in a predetermined manner. The interaction of the optical beams generated by the LEDs of the position sensor 91 generates a signal that indicates the longitudinal position of the optical flag 206 relative to the position sensor 91 with a substantially high degree of resolution. The resolution of the position sensor 91 may be about 200 to about 400 cycles per inch, specifically, about 350 to about 370 cycles per inch. The position sensor 91 may have a speed response time (position/time resolution) of 0 to about 120,000 Hz, where one dark and light stripe of the flag constitutes one Hertz, or cycle per second. The position of the optical encoder flag 206 relative to the magnetic member 102, driver coil pack 88 and position sensor 91 is such that the optical encoder 91 can provide precise positional information about the penetrating member 83 over the entire length of the penetrating member's power stroke.

An optical encoder that is suitable for the position sensor 91 is a linear optical incremental encoder, model REDS 9200, manufactured by Agilent Technologies. The model REDS 9200 may have a length of about 20 to about 30 mm, a width of about 8 to about 12 mm, and a height of about 9 to about 11 mm. Although the position sensor 91 illustrated is a linear optical incremental encoder, other suitable position sensor embodiments could be used, provided they posses the requisite positional resolution and time response. The HEDS 9200 is a two channel device where the channels are 90 degrees out of phase with each other. This results in a resolution of four times the basic cycle of the flag. These quadrature outputs make it possible for the processor to determine the direction of penetrating member travel. Other suitable position sensors include capacitive encoders, analog reflective sensors, such as the reflective position sensor discussed above, and the like.

A coupler shaft guide 111 is disposed towards the proximal end 81 of the body fluid sampling device 80. The guide 111 has a guide lumen 112 disposed in the guide 111 to slidingly accept the proximal portion 92 of the elongate coupler shaft 84. The guide 111 keeps the-elongate coupler shaft 84 centered horizontally and vertically in the slot 102 of the optical encoder 91.

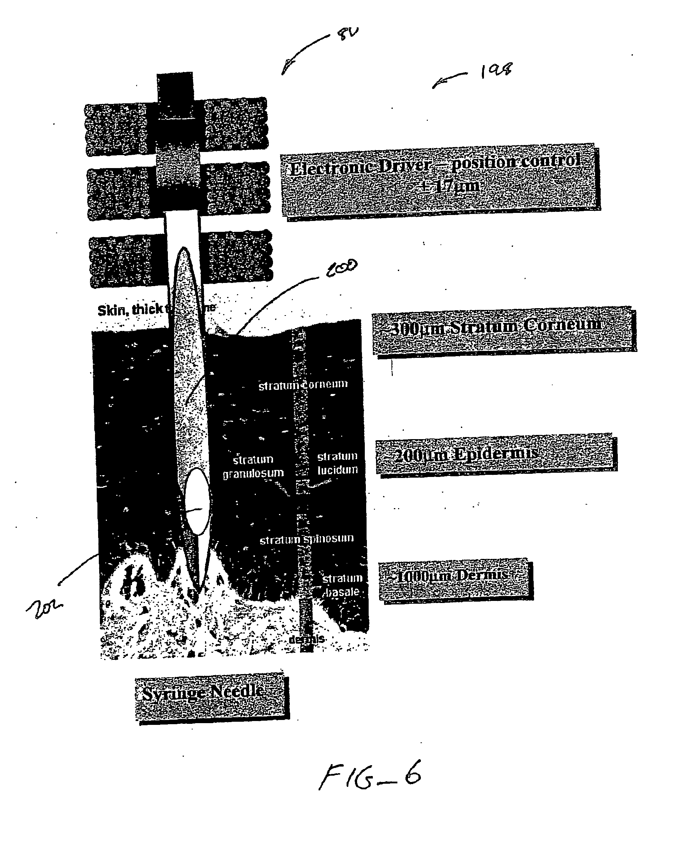

Referring now to Figure 6, one embodiment for a device for fluid sampling is shown. As seen, a patent needle device 198 for accurately positioning a patent needle 200 within the skin layer is shown. The needle 200 has an opening 202 for receiving fluid from the patient.

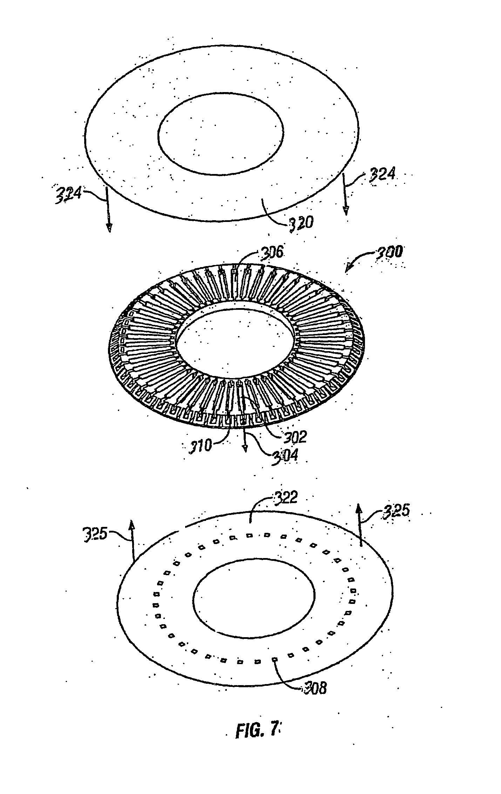

Referring now to Figure 7, a still further embodiment of a cartridge according to the present invention will be described. Figure 7 shows one embodiment of a cartridge 300 which may be removably inserted into an apparatus for driving penetrating members to pierce skin or tissue. The cartridge 300 has a plurality of penetrating members 302 that may be individually or otherwise selectively actuated so that the penetrating members 302 may extend outward from the cartridge, as indicated by arrow 304, to penetrate tissue. In the present embodiment, the cartridge 300 may be based on a flat disc with a number of penetrating members such as, but in no way limited to, (25, 50, 75, 100, ...) arranged radially on the disc or cartridge 800. It should be understood that although the cartridge 300 is shown as a disc or a disc-shaped housing, other shapes or configurations of the cartridge may also work without departing from the spirit of the present invention of placing a plurality of penetrating members to be engaged, singly or in some combination, by a penetrating member driver.

Each penetrating member 302 may be contained in a cavity 306 in the cartridge 300 with the penetrating member's sharpened end facing radially outward and may be in the same plane as that of the cartridge. The cavity 306 may be molded, pressed, forged, or otherwise formed in the cartridge. Although not limited in this manner, the ends of the cavities 306 may be divided into individual fingers (such as one for each cavity) on the outer periphery of the disc. The particular shape of each cavity 306 may be designed to suit the size or shape of the penetrating member therein or the amount of space desired for placement of the analyte detecting members 808. For example and not limitation, the cavity 306 may have a V-shaped cross-section, a U-shaped cross-section, C-shaped cross-section, a multi-level cross section or the other cross-sections. The opening 810 through which a penetrating member 302 may exit to penetrate tissue may also have a variety of shapes, such as but not limited to, a circular opening, a square or rectangular opening, a U-shaped opening, a narrow opening that only allows the penetrating member to pass, an opening with more clearance on the sides, a slit, a configuration as shown in Figure 75, or the other shapes.

In this embodiment, after actuation, the penetrating member 302 is returned into the cartridge and may be held within the cartridge 300 in a manner so that it is not able to be used again. By way of example and not limitation, a used penetrating member may be returned into the cartridge and held by the launcher in position until the next lancing event. At the time of the next lancing, the launcher may disengage the used penetrating member with the cartridge 300 turned or indexed to the next clean penetrating member such that the cavity holding the used penetrating member is position so that it is not accessible to the user (i.e. turn away from a penetrating member exit opening), In some embodiments, the tip of a used penetrating member may be driven into a protective stop that hold the penetrating member in place after use. The cartridge 300 is replaceable with a new cartridge 300 once all the penetrating members have been used or at such other time or condition as deemed desirable by the user.

Referring still to the embodiment in Figure 7, the cartridge 300 may provide sterile environments for penetrating members via seals, foils, covers, polymeric, or similar materials used to seal the cavities and provide enclosed areas for the penetrating members to rest in. In the present embodiment, a foil or seal layer 320 is applied to one surface of the cartridge 300. The seal layer 320 may be made of a variety of materials such as a metallic foil or other seal materials and may be of a tensile strength and other quality that may provide a sealed, sterile environment until the seal layer 320 is penetrate by a suitable or penetrating device providing a preselected or selected amount of force to open the sealed, sterile environment. Each cavity 306 may be individually sealed with a layer 320 in a manner such that the opening of one cavity does not interfere with the sterility in an adjacent or other cavity in the cartridge 800. As seen in the embodiment of Figure 7, the seal layer 320 may be a planar material that is adhered to a top surface of the cartridge 800.

Depending on the orientation of the cartridge 300 in the penetrating member driver apparatus, the seal layer 320 maybe on the top surface, side surface, bottom surface, or other positioned surface. For ease of illustration and discussion of the embodiment of Figure 7, the layer 320 is placed on a top surface of the cartridge 800. The cavities 306 holding the penetrating members 302 are sealed on by the foil layer 320 and thus create the sterile environments for the penetrating members. The foil layer 320 may seal a plurality of cavities 306 or only a select number of cavities as desired.

In a still further feature of Figure 7, the cartridge 300 may optionally include a plurality of analyte detecting members 308 on a substrate 822 which may be attached to a bottom surface of the cartridge 300. The substrate may be made of a material such as, but not limited to, a polymer, a foil, or other material suitable for attaching to a cartridge and holding the analyte detecting members 308. As seen in Figure 7, the substrate 322 may hold a plurality of analyte detecting members, such as but not limited to, about 10-50, 50-100, or other combinations of analyte detecting members. This facilitates the assembly and integration of analyte detecting members 308 with cartridge 300. These analyte detecting members 308 may enable an integrated body fluid sampling system where the penetrating members 302 create a wound tract in a target tissue, which expresses body fluid that flows into the cartridge for analyte detection by at least one of the analyte detecting members 308. The substrate 322 may contain any number of analyte detecting members 308 suitable for detecting analytes in cartridge having a plurality of cavities 306. In one embodiment, many analyte detecting members 308 may be printed onto a single substrate 322 which is then adhered to the cartridge to facilitate manufacturing and simplify assembly. The analyte detecting members 308 may be electrochemical in nature. The analyte detecting members 308 may further contain enzymes, dyes, or other detectors which react when exposed to the desired analyte. Additionally, the analyte detecting members 308 may comprise of clear optical windows that allow light to pass into the body fluid for analyte analysis. The number, location, and type of analyte detecting member 308 may be varied as desired, based in part on the design of the cartridge, number of analytes to be measured, the need for analyte detecting member calibration, and the sensitivity of the analyte detecting members. If the cartridge 300 uses an analyte detecting member arrangement where the analyte detecting members are on a substrate attached to the bottom of the cartridge, there may be through holes (as shown in Figure 76), wicking elements, capillary tube or other devices on the cartridge 300 to allow body fluid to flow from the cartridge to the analyte detecting members 308 for analysis. In other configurations, the analyte detecting members 308 may be printed, formed, or otherwise located directly in the cavities housing the penetrating members 302 or areas on the cartridge surface that receive blood after lancing.

The use of the seal layer 320 and substrate or analyte detecting member layer 822 may facilitate the manufacture of these cartridges 10. For example, a single seal layer 320 may be adhered, attached, or otherwise coupled to the cartridge 300 as indicated by arrows 324 to seal many of the cavities 306 at one time. A sheet 322 of analyte detecting members may also be adhered, attached, or otherwise coupled to the cartridge 300 as indicated by arrows 325 to provide many analyte detecting members on the cartridge at one time. During manufacturing of one embodiment of the present invention, the cartridge 300 may be loaded with penetrating members 302, sealed with layer 320 and a temporary layer (not shown) on the bottom where substrate 322 would later go, to provide a sealed environment for the penetrating members. This assembly with the temporary bottom layer is then taken to be sterilized. After sterilization, the assembly is taken to a clean room (or it may already be in a clear room or equivalent environment) where the temporary bottom layer is removed and the substrate 322 with analyte detecting members is coupled to the cartridge as shown in Figure 7. This process allows for the sterile assembly of the cartridge with the penetrating members 302 using processes and/or temperatures that may degrade the accuracy or functionality of the analyte detecting members on substrate 322. As a nonlimiting example, the entire cartridge 300 may then be placed in a further sealed container such as a pouch, bag, plastic molded container, etc...to facilitate contact, improve ruggedness, and/or allow for easier handling,

In some embodiments, more than one seal layer 320 may be used to seal the cavities 306. As examples of some embodiments, multiple layers may be placed over each cavity 306, half or some selected portion of the cavities may be sealed with one layer with the other half or selected portion of the cavities sealed with another sheet or layer, different shaped cavities may use different seal layer, or the like. The seal layer 320 may have different physical properties, such as those covering the penetrating members 302 near the end of the cartridge may have a different color such as red to indicate to the user (if visually inspectable) that the user is down to say 10, 5, or other number of penetrating members before the cartridge should be changed out.

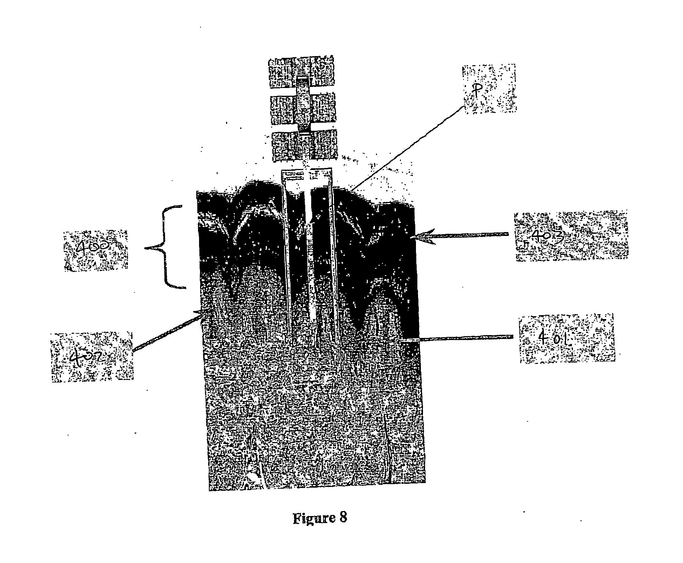

Referring now to Figure 8, the anatomy of the finger will be described in further detail. Figure 8 shows the ideal position of penetrating member in skin. The capillaries are located in the epidermis 400, so that the ideal position of the penetrating member would be at the junction of the stratum papilare 401 and the superficial reticular layer 402, if enough blood could be elicited from this shallow lancing depth Figure 8 (penetrating member not shown to scale). In one embodiment of the present invention, it has been shown that enough blood for sample acquisition and analyte detection can be obtained from lancing 300 micrometers below the surface of the stratum corneum 403 using a 300 micrometer diameter penetrating member. On average 300 - 500 nL of blood is obtained which is enough to fill analyte detecting members requiring 100 - 200 nL of blood for analyte detection. This results in a painless blood sampling event as few nerves and tissue are cut as compare to going deeper, which is commonly the case with mechanically actuated penetrating members as patients tend to set these with greater depth penetration than required to ensure a blood sample every lancing event.

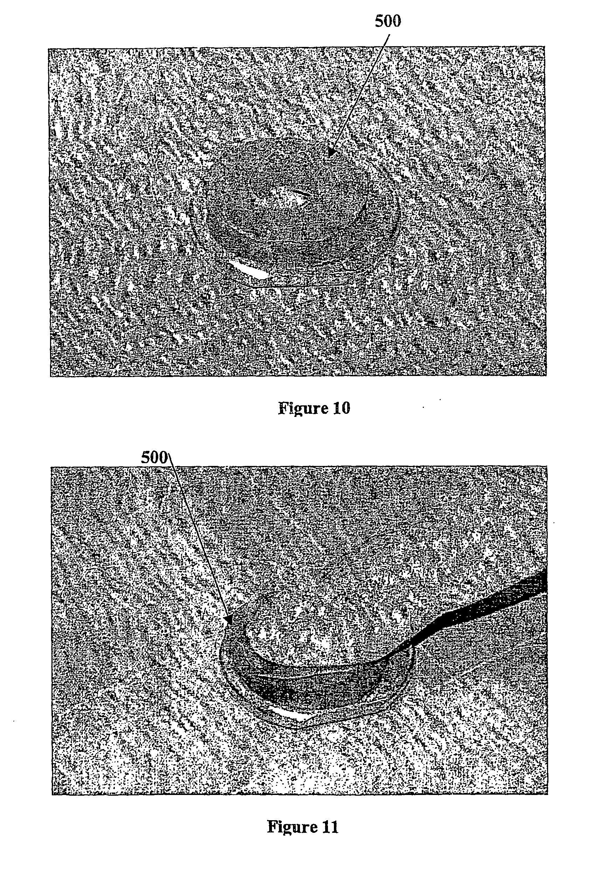

To lance, the patient places the finger in the cradle or support in a relaxed way as shown starting in Figure 10, without applying pressure. The lancing event occurs while the finger is relaxed. The correct depth of penetration is accounted for by the contact point routine implicit in the lancing algorithm, which determines where the skin is relative to the displacement of the penetrating member from zero position. Once the lancing event has occurred, the patient presses the finger against the surface to extract the blood. The mechanisms described herein in teach automated protocols for lancing in a relaxed and then pressured mode.

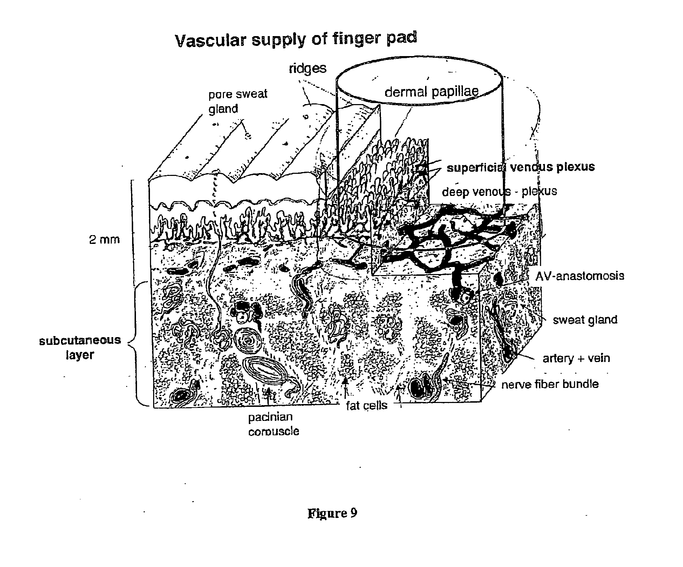

Referring to Figure 9, the anatomical rationale for such a lancing protocol is as follows. When the finger is relaxed and lancing takes place, blood flows from the arterial system to the capillaries and then out through the venous plexus. After lancing, pressing the finger to the cradle prevents the venous blood escaping into the venous plexus because the subcutaneous veins are shut off. The arterial flow is not affected by this pressure (the arterial pressure is 40 - 60 mmHg and the venous about 10 mmHg). Thus, the blood supply to the capillary net is guaranteed. As blood flows into the capillary net it is prevented from escaping through the venous plexus, and as the pressure builds up the blood is forced out through the wound channel since the elastic fibers of the venous vessels are functionally linked to the elastic fiber net if the dermis. A cylinder shaped volume of blood (shown in red) will be available for expressing up through the wound channel (if the front end is appropriately designed).

Prepressing the finger prior to lancing puts the elastic system under stress, and the vascular system will collapse after lancing due to rearrangement of the elastic net, which now impairs blood flow rather than assisting in pushing the blood up through the wound channel. Hence, any of the se methods applying a tourniquet before lancing will not have the desired result.

Referring now to Figure 10, one embodiment not included in the present invention will now be described in further detail. The front end of a penetrating member device is the primary device-tissue interface. Its purpose, in this embodiment, is to provide a location in space for the skin surface to rest against and to stabilize the skin during lancing. The front end can be designed to be interactive; to aid in the expression of a usable blood sample from a penetrating member wound. The mechanisms may allow for lancing in a relaxed and then pressured mode.

The following describes several designs which include some but no necessarily all of the following:
1. Provide a place for a patient to press a finger
2. Co-located with the lancing mechanism, such that a penetrating member can pass unobstructed through the front-end and lance the finger pressed against it
3. Hold the patient's finger in a stationary position
4. Convert the force applied by the patient's finger into a pressure on the finger which increases the blood flow out of the penetrating member hole after lancing
5. Do not use electrical power to do any work

Figures 10 and 11 show one embodiment not included in the present invention. The embodiment in Figure 10 is an annular finger pad 500 of around 1" outer diameter, 3/8" inner diameter, and ¼" thickness. Figure 11 shows a finger pushing on the pad 500. This pad 500 may be made of a visco-elastic gel, and thus applies a more even distribution of pressure to the finger than a standard elastomer. The elastomeric gel material is of very low hardness. Application of pressure by the fingertip (or other body part) causes the material to deform. A component of this deformation is the expansion of the material in a direction perpendicular to the direction of applied pressure. The inner diameter of the ring 500 decreases under this pressure and applies a lateral pressure to the portion of the finger that is located in the center of the ring. The pad 500 is also constrained within a rigid circular support such that the surface of the patient's finger is prevented from moving laterally once pressure is applied. This pad 500 may be formed about a penetrating member exit port so as to position the finger or other tissue for lancing. The force applied by the user may be turned into a "milking" pressure.

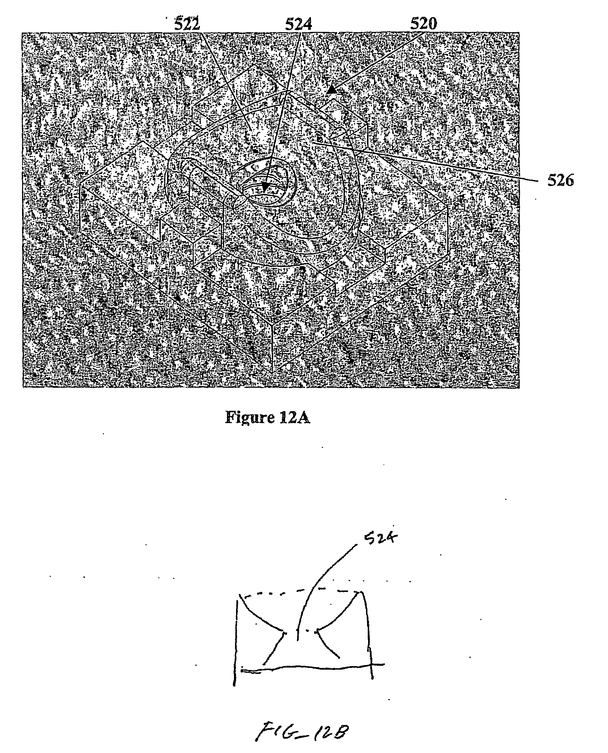

Referring now to Figure 12A, an embodiment of the present invention will now be described. A semi-cylindrically shaped pad 520 is shown that conforms to the side of the finger. The surface 522 of the pad 520 may be curved with a portion extending upward away from the opening 524. This allows tissue away from the wound site to be engaged. Although not limited to the following, it may be made of a visco-elastic gel. The shape of pad 520 allows for easy lancing of the side of the finger. Through the bottom of the pad may be a tapered hole, through which a penetrating member can pass through. When finger pressure is applied, the side walls 526 of the hole and of the cylinder collapse inward and press against the finger, which supplies a milking force. The opening 522 may be between 6mm and 10mm. In one embodiment, it is 8mm.

Figure 12B is a cross-section and shows that opening 524 leads to an area with a tapered profile. This optional feature further allows the sides to collapse downward and provide greater flexibility when the finger or tissue presses on the device.

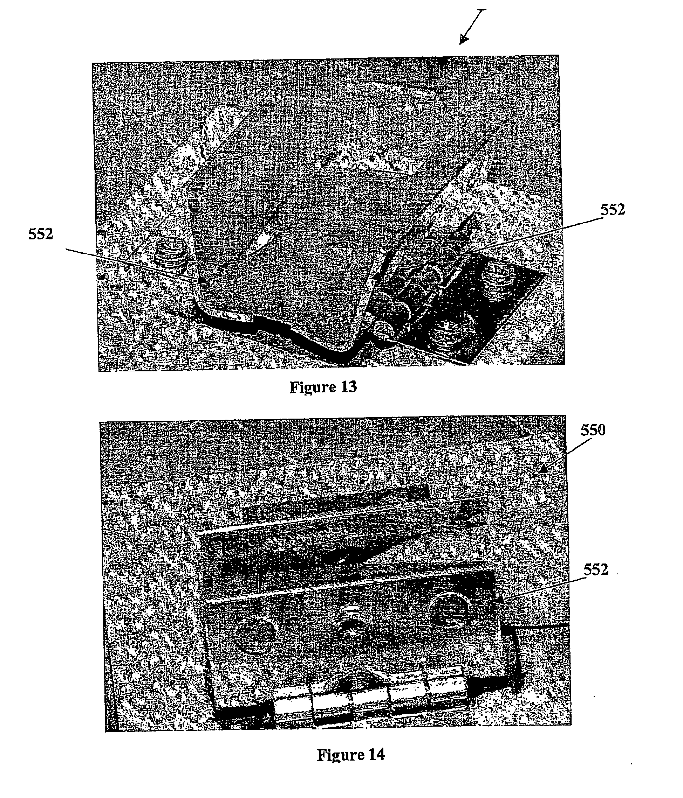

Referring now to Figures 13 and 14, a still further embodiment not included in the present invention will now be described. With a visco-elastic gel forming an open channel into which a finger can be inserted, and with a hole in the bottom for passage of a penetrating member this tissue interface 550 also allows for pressure to be applied at areas away from the wound site. In this particular embodiment, on either side of the gel structure (parallel with the channel) are placed two rigid "L" shaped structures 552. It should be understood that other shaped structures such as C-shaped, U-shaped, V-shaped, or the like may also be used to apply force at positions away from the wound site. These shapes may be cross-sectional shapes. These are hinged at the vertices of the "L" such that if a downward force is applied to the bottom surface of the gel, the "L" structures collapse inward and close off the top of the gel channel. As seen in Figure 14, if a finger were to be placed in the channel and a downward force applied, the gel would squeeze the top surface of the finger and increase the blood flow out of the penetrating member wound. There is thus a linkage between pressure on a bottom contact portion and the amount of pressure applied along the side walls and/or top walls to the finger.

This design can also be used with a mechanical release. Before lancing, the hinges can be prevented from swiveling. After lancing, the hinges are released and will rotate due to the finger pressure. This will cause a squeezing of the top of the finger, but after lancing has taken place.

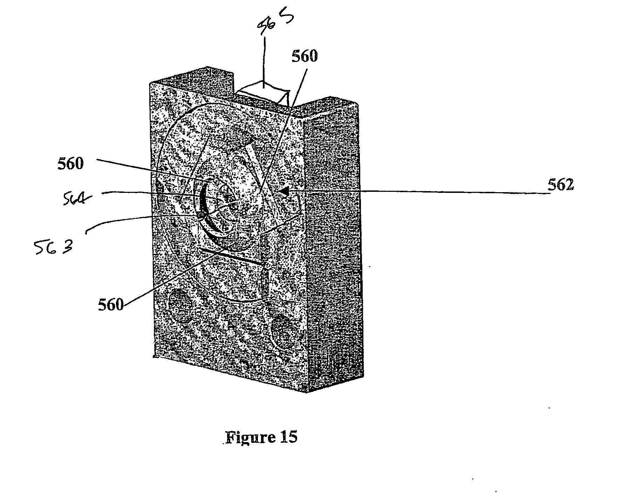

Referring now to the embodiment of Figure 15, a similar design to that of Figures 13 and 14 Hinge is used, except that three rigid structures 560 are arranged around a central point instead of two parallel structures arranged on either side of a central line. When pressure is applied to the bottom surface, the bottom surface of each of these structures rotates downward, and the vertical side rotates radially inward and presses against the surface of the finger. A hinge 562 allows each of portions 560 to articulate and flex. In some embodiments, the hinge 562 may be cutaway or weakened portion to allow for movement. These rotating structures can be large enough to encompass the entire tip of the finger, or in some embodiments, may be smaller and affect only the skin near the lancing site. Furthermore, a gel surface pad is optionally available and may be added to 560. The radially inward pressure supplied by the rotated structures increases blood flow out of the penetrating member wound. This design includes additional structures at the central point. These structures contact the apex of the skin contour and serve to stabilize the skin nearest to the lancing site. As seen in Figure 15, the finger may pouch inside the area 563. This allows the elements 560 to press on tissue above the area that has pooched in the area 563. Some embodiments may have the user contact a center O-ring area 564. The elements 560 together will form a ring when pressed on by the user.

As with the design of Figure 13, this design can be used with a release mechanism 565 that only allows for the rotation of the structures and squeezing of the finger after lancing has taken place. This presses blood from the top of the finger towards the exit wound. The release mechanism 565 may be a lock that slides to release the elements 560 and allow them the flex about the hinge. Otherwise, the release mechanism 565 supports the back sides of the elements 560 and prevent them from flexing inwards.

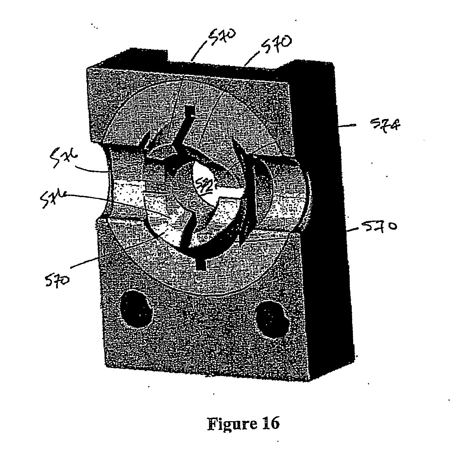

Referring now to Figure 16, yet another embodiment not included in the present invention is shown. In this embodiment, a device with four movable elements 570 is shown. Similar to the embodiment of Figure 15, these elements 570 may be hinged to move downward when pressed. The gaps 571 between the elements 570 allow for movements of the elements when a finger or other part presses on them. The opening 572 is located in the center, and it is where the penetrating member will engage tissue. The elements 570 may also be shaped to allow a side of the finger to fit onto the interface. In this embodiment, this is the larger cut outs 574 that allow a side of a finger to fit. This sized to apply pressure closer to the wound site. Figure 16 shows that there is a bottom surface 574 and then side or top surfaces 576 that will press on the finger when a bottom surface is moved. This embodiment may optionally use a release mechanism described above for the embodiment in Figure 15.

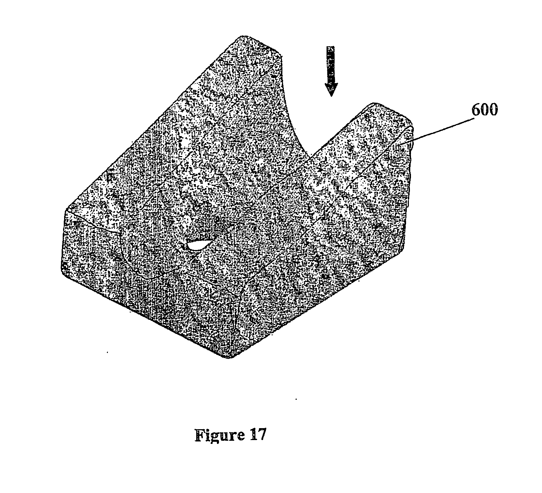

Referring to Figure 17, a still further embodiment not included in the present invention will now be described. This is similar to the embodiment of Figure 12, except that one end 600 of the gel extends vertically much higher than the other. The patient's finger is inserted such that the finger centerline is parallel to the base and the high end of the gel is on the palm side (as opposed to the tip side) of the finger. Therefore, as the finger fits into the slot in the gel (which is slightly narrower than the finger), the side walls of the gel will press against the rear portion of the finger first and force blood towards the tip of the finger. The penetrating member hole is offset towards the tip end of the device, and so lancing takes place into a blood enriched section of the finger.

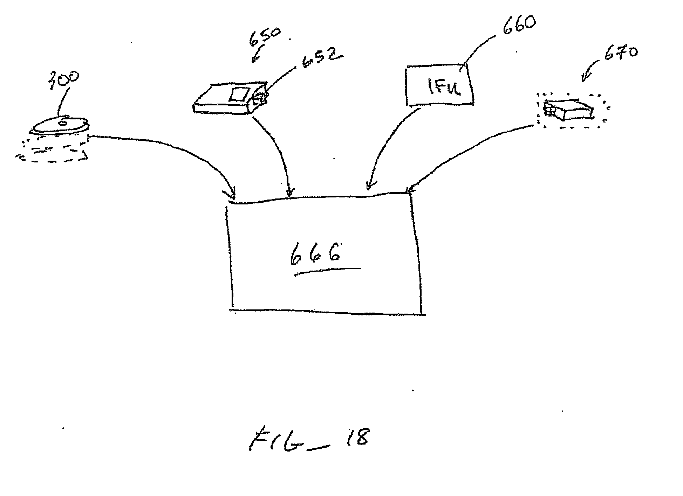

Figure 18 shows a kit according to one embodiment having a device 650 wherein the tissue interface is a front end 652 on a body fluid sampling device. The body fluid sampling device may include a front end 652 similar to any of those described herein. The instructions for use (IFU) 660 may tell the user to apply no or minimal pressure against the device during lancing and applying pressure post lancing for spontaneous blood generation. All of the above may be housed in a container or pouch 666. One or more disc shaped cartridges such as but not limited to that described in Figure 7 may also be included. Other embodiments may have a cassette 670 with a front end 652. the cassette 670 may house the disc shaped cartridge 300 therein.

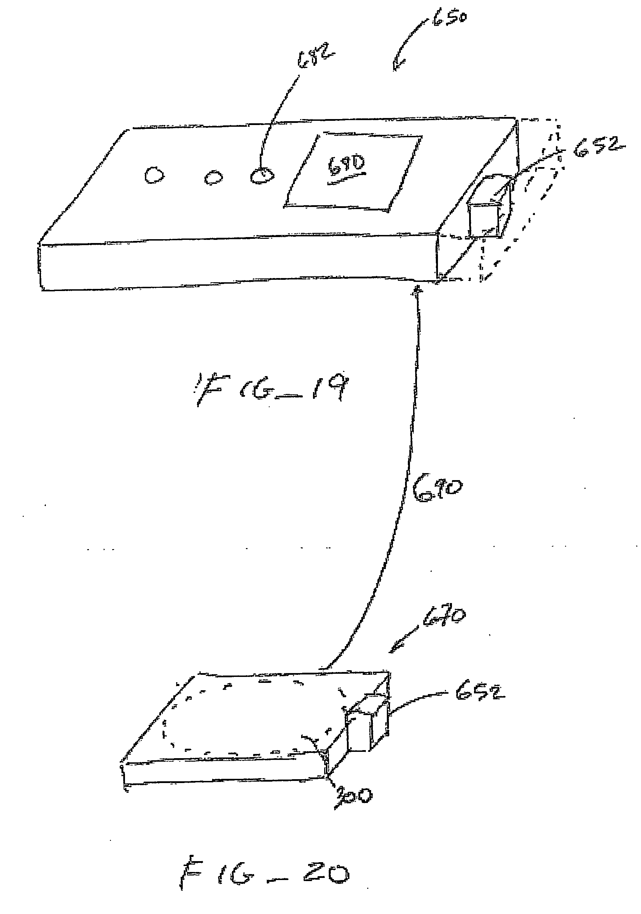

Figure 19 shows that the front end maybe formed on the body fluid sampling device 650. The device 650 may include a display 680 and various buttons 682 for controlling the device and may use an electronic penetrating member driver as described herein. The body fluid sampling device may include a front end 652 similar to any of those described herein. The front end 652 may be removably coupled to the housing 654 on the device 650. This may allow for ease of cleaning or replacement. The front end 652 may be adhered, clamped, gripped, or otherwise attached to the housing. In some embodiments, the front end 652 may be recessed inside the housing as indicated by the phantom lines 688.

Figure 20 shows a still further embodiment where a cassette 670 houses the cartridge 300 and may have the front end 652 attached to the cassette 670 and not directly to the device 650. The cassette 670 may be designed to fit inside the device 650 as indicated by arrow 690. The front end 652 may protrude outward from the housing of device 650 or be recessed (fully or partially).

While the invention has been described and illustrated with reference to certain particular embodiments thereof, those skilled in the art will appreciate that various adaptations, changes, modifications, substitutions, deletions, or additions of procedures and protocols may be made. For example, with any of the above embodiments, the tissue interface may be used with any sample capture mechanism. The tissue interface may formed on a housing for the tissue sampling device or it may be formed on the disposable such as but not limited to the lancing cartridge.

The publications discussed or cited herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Expected variations or differences in the results are contemplated in accordance with the objects and practices of the present invention. It is intended, therefore, that the invention be defined by the scope of the claims which follow and that such claims be interpreted as broadly as is reasonable.

Aspects not included in the invention are defined by the following independent and dependent numbered clauses.
1. A device for assisting fluid sampling comprising:
   a tissue interface having a tissue interface surface configured to improve fluid flow from a wound site.
2. A device for assisting fluid sampling comprising:
   a tissue interface having a tissue interface surface; and
   wherein the surface is shaped to provide pressure on the tissue, except at one location;
   said tissue interface made of a pliable material;
   said tissue interface apply an even amount of pressure to the tissue that is engaged to facilitate fluid sampling.
3. The device of clause 1 wherein said is a viscoelastic gel.
4. The device of clause 1 wherein tissue interface surface is made of a polyurethane.
5. The device of clause 1 wherein said interface allows for no pressure during lancing and applying pressure post lancing for spontaneous blood generation.
6. The device of clause 1 wherein said tissue surface is curved to engage tissue away from a wound site created by a penetrating member.
7. The device of clause 1 having side walls that come up away from where a penetrating member opening is to allow pressure to be applied at a site away from the wound site.
8. The device of clause 1 allows for at least 1 mm of travel, to allow the user to push against the tissue interface surface to create pressure against other areas of the tissue away from the wound.
9. The device of clause 1 allows for at least 2 mm of travel, to allow the user to push against the tissue interface surface to create pressure against other areas of the tissue away from the wound.
10. The device of clause 1 wherein the tissue interface is a front end on a body fluid sampling device.
11. A kit comprising:
   a device of clause 1 wherein the tissue interface is a front end on a body fluid sampling device;
   a body fluid sampling device, wherein said device of clause 1 is mounted on the body fluid sampling device;
   instructions for use instructing the user to apply no or minimal pressure against the device of clause 1 during lancing and applying pressure post lancing for spontaneous blood generation.
12. A device for assisting fluid sampling comprising:
   a tissue interface having a tissue interface surface; and
   said surface curved to provide pressure on the tissue, except at one location;
   said surface having a side portion for engaging tissue located away from the lancing site;
   said tissue interface made of a pliable material;
   said tissue interface apply an even amount of pressure to the tissue that is engaged to facilitate fluid sampling.
13. The device of clause 12 allowing a user to provide no pressure during lancing, applying pressure post lancing for spontaneous blood generation
14. The device of clause 12 wherein said tissue interface surface is shaped so that the tissue can be engaged without tourniquet before lancing has adverse effect on blood extraction from wound.
15. The device of clause 12 further comprising a plurality of hinge based designs are unique way to translate the direction of force
16. The device of clause 12 further comprising a plurality of tissue surfaces shaped the designs to produce a radially divergent force in the proximity of the wound channel, this helps the wound channel stay open.
17. A method for assisting fluid sampling comprising:
   providing a tissue interface having a tissue interface surface;
   piercing the tissue with a penetrating member while providing a first pressure on the tissue interface surface; and
   after lancing, having the user press on the tissue interface to provide a second pressure on the tissue, except at one location on the tissue to allow for spontaneous fluid generation from a wound created on user tissue.
18. A method for assisting fluid sampling comprising:
   providing a tissue interface having a tissue interface surface;
   piercing the tissue with a penetrating member while providing a first pressure on the tissue interface surface; and
   after lancing, having the user press on the tissue interface to provide a second pressure on the tissue, except at one location to allow for spontaneous fluid generation from a wound created on user tissue.
19. A device comprising:
   a housing;
   an electronic penetrating member driver;
   a tissue interface coupled to the housing, wherein the interface has a surface configured to improve fluid flow from a wound created on a patient;
   said surface curved to provide pressure on the tissue, except at one location;
   said surface having a side portion for engaging tissue located away from the lancing site;
   said tissue interface made of a pliable material;
   said tissue interface apply an even amount of pressure to the tissue that is engaged to facilitate fluid sampling.
20. The device of clause 19 further comprising a disc shaped cartridge having a plurality of penetrating members.

## Claims

1. A device for assisting fluid sampling comprising:
a penetrating member (83) operable to penetrate tissue of a finger at a lancing site during a lancing event to create a wound channel;
a tissue interface having an opening (524) through which the penetrating member can pass and a tissue interface surface (522) for a skin surface to rest against and stabilize during lancing;
**characterized in that** the tissue interface comprises a semi-cylindrically shaped pad (520), wherein the tissue interface surface is curved and wherein the pad comprises side walls (526) extending upward and away from the opening for engaging tissue away from the wound channel and applying a milking force to the tissue when pressure is applied to the tissue interface.

2. The device of claim 1, wherein the opening comprises a tapered hole.

3. The device of claim 2, wherein side walls of the hole and the side walls of the pad collapse inward and press against the tissue.

4. The device of any preceding claim, wherein the pad is formed of a pliable material.

5. The device of claim 4, wherein the pad is formed of a visco-elastic gel.

## Patentansprüche

1. Vorrichtung zur Unterstützung einer Fluidprobenahme, umfassend:
ein Durchdringungselement (83), das zum Durchdringen von Gewebe eines Fingers an einer Einstechstelle während eines Einstechvorgangs zur Erzeugung eines Wundkanals betätigt werden kann,
ein Gewebe-Zwischenelement mit einer Öffnung (524), durch die das Durchdringungselement hindurchtreten kann, und einer Gewebe-Zwischenelementoberfläche (522) für eine Hautoberfläche, die als Auflage während des Einstechens und zur Stabilisierung während des Einstechens dient,
**dadurch gekennzeichnet, dass** das Gewebe-Zwischenelement ein halbzylindrisch geformtes Kissen (520) umfasst, wobei die Gewebe-Zwischenelementoberfläche gekrümmt ist und wobei das Kissen Seitenwände (526), die sich aufwärts und weg von der Öffnung erstreckt, zum Ineingriffnehmen von Gewebe entfernt von dem Wundkanal und Ausüben einer Saugkraft auf das Gewebe, wenn auf das Gewebe-Zwischenelement Druck ausgeübt wird, umfasst.

2. Vorrichtung nach Anspruch 1, bei der die Öffnung ein kegelförmiges Loch umfasst.

3. Vorrichtung nach Anspruch 2, bei der sich Seitenwände des Lochs und die Seitenwände des Kissens nach innen wölben und gegen das Gewebe drücken.

4. Vorrichtung nach einem der vorhergehenden Ansprüche, bei der das Kissen aus einem biegsamen Material ausgebildet ist.

5. Vorrichtung nach Anspruch 4, bei der das Kissen aus einem viskoelastischen Gel ausgebildet ist.

## Revendications

1. Dispositif pour faciliter l'échantillonnage d'un fluide comprenant :
un élément de pénétration (83) pouvant opérer de manière à pénétrer dans le tissu d'un doigt au niveau d'un site de piqûre durant un événement de piqûre pour créer un canal de plaie ;
une interface tissulaire présentant une ouverture (524) à travers laquelle l'élément de pénétration peut passer et une surface d'interface tissulaire (522) pour qu'une surface de peau repose contre celle-ci et soit stabilisée durant la piqûre ;
**caractérisé en ce que** l'interface tissulaire comprend un tampon de forme semi-cylindrique (520), dans lequel la surface d'interface tissulaire est incurvée, et dans lequel le tampon comprend des parois latérales (526) s'étendant vers le haut en s'éloignant de l'ouverture pour engager un tissu à distance du canal de plaie et appliquer une force de traite au tissu quand une pression est appliquée à l'interface tissulaire.

2. Dispositif selon la revendication 1, dans lequel l'ouverture comprend un trou conique.

3. Dispositif selon la revendication 2, dans lequel les parois latérales du trou et les parois latérales du tampon s'affaissent vers l'intérieur et s'appuient contre le tissu.

4. Dispositif selon l'une quelconque des revendications précédentes, dans lequel le tampon est formé d'un matériau pliable.

5. Dispositif selon la revendication 4, dans lequel le tampon est formé d'un gel viscoélastique.
